(19) Europäisches Patentamt
European Patent Office
Office européen des brevets

(11) Publication number: **0 202 661 B1**

(12) **EUROPEAN PATENT SPECIFICATION**

(45) Date of publication of patent specification: **04.08.93** (51) Int. Cl.⁵: **A61K 31/435**

(21) Application number: **86106863.3**

(22) Date of filing: **21.05.86**

Divisional application 92115805.1 filed on 21/05/86.

The file contains technical information submitted after the application was filed and not included in this specification

# (54) Isolation of castanospermine and its use as an antidiabetic agent.

(30) Priority: **24.05.85 US 738127**

(43) Date of publication of application:
**26.11.86 Bulletin 86/48**

(45) Publication of the grant of the patent:
**04.08.93 Bulletin 93/31**

(84) Designated Contracting States:
**AT BE CH DE FR GB IT LI LU NL SE**

(56) References cited:

**FED. PROC.,vol. 43, no. 6, Congress June 3-7, 1984, page 1554, ab- stract 804; r. saul et al: "Mechanism of action and in vivo studies of castano- spermine inhibition of alpha-glucosidase"**

**FED. PROC.,vol. 43, no. 3, 1984, page 618, abstact 1946; L. GHIDUNI et al: "Introduction of a Type II glycogenosis-like state by intra-peritopeal injection of castanospermine"**

(73) Proprietor: **MERRELL DOW PHARMACEUTICALS INC.**
**2110 East Galbraith Road**
**Cincinnati Ohio 45215-6300(US)**

(72) Inventor: **Liu, Paul S.**
**7320 Lakeside Drive**
**Indianapolis Indiana 46278(US)**
Inventor: **Rhinehart, Barry L.**
**9021 Jackson**
**Indianapolis Indiana 46231(US)**

(74) Representative: **Sgarbi, Renato et al**
**GRUPPO LEPETIT S.p.A. Patent and Trademark Department Via Roberto Lepetit, 34**
**I-21040 Gerenzano (Varese) (IT)**

**EP 0 202 661 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**FED: PROC., vol. 42, no. 47, 1983, page 2083, abstract 1908; R. SAUL et al: "Castanospermine , a tetrahydroxylated alkaloid that inhibits alpha and beta-glucosidases"**

**PROC. NATL. ACAD. SCI. USA, vol. 82, no.1, January 1985, pages93- 97 R. SAUL et al: Castanospermine inhibits alpha-glucosidase activities and alters glycogen distribution in animals"**

**METABOLISM, vol. 31, no. 7, July 1982, pages 710-714, Grune and Stratton, Inc.; H.P. KRAUSE et al: "Reduction of carbohydrate-induced hypertriglyceridemia in (fa,fa) "Zucker" rats by the alpha-glucosidase inhibitor acarbose (BAY g 5421)"**

**METABOLISM, vol. 31, no. 1, January 1982, pages 88-92, Grune & Stratton, Inc.; R. STUART et al: "Effect of a glucosidase inhibitor on the metabolic response of diabetic rats to a high carbohydrate diet, consisting of starch and sucrose, or glucose**

**PATENT ABSTRACTS OF JAPAN, vol. 11, no. 69 (C-407) (2516) March 3, 1987 &JP-A-61 227 566 (FUJISAWA PHARMACEUTICAL CO. LTD.) 09.10.1986**

**ARTERIOSCLEROSIS, vol. 5, no. 5, September/October 1985, page 542A; E.H. EDWARDS et al: "Effect of glycoprotein processing inhibitors on LDL meta- bolism in aortic smooth muscle cells"**

**LIFE SCIENCES, vol. 39, no. 7, August 18, 1986, pages 645-650, Pergamon Journals, US; A.M. SCOFIELD et al: "Inhibition of mammalian digestive disaccharidases by polyhydroxy alkaloids"**

**PHYTOCHEMISTRY, vol. 20, no. 4, 1981, pages 811-814, Pergamon Press Ltd., GB L.D. HOHENSCHUTZ et al:"Castanospermine, A 1,6,7,8-tetrahydroxyoctahydroindol- izine, from seeds of Castanospermum Australe"**

## Description

Castanospermine is an alkaloid having the following formula:

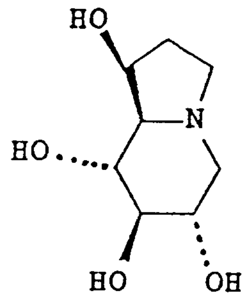

Systematically, this compound is named (1S,6S,7R,8R,8aR)-1,6,7,8-tetrahydroxyindolizidine.

The isolation of this compound from Castanospermum australe has been described by L.D. Hohenshutz, et al., Phytochemistry, 20, 811 (1981). The procedure involves a tedious extraction and a subsequent washing with pyridine and only small quantities of the desired product are obtained. R.C. Bernotas, et al., Tetrahedron Letters, 25, 165 (1984) obtained castanospermine by total synthesis and thereby established the absolute configuration of the compound.

Several publications have appeared which describe castanospermine as an inhibitor of a number of plant enzymes. See Y.T. Pan, et al., Biochemistry, 22, 3975 (1983); R. Saul, et al., Archives of Biochemistry and Biophysics, 221, 593 (1983); R. Saul, et al., Archives of Biochemistry and Biophysics, 230, 668 (1984). More recently, Saul et al., Proc. Nat'l. Acad. Sci. USA, 82, 93 (1985) have indicated that castanospermine inhibits intestinal glycosidases (i.e., maltase and sucrase) in rats when administered by injection and that diarrhea and high levels of intestinal bacterial flora were observed when high doses were administered. However, they gave no indication that castanospermine would be useful for any purposes as a result of these facts.

Acarbose, or Bay-g-5421, is described in ***Metabolism***, Vol. 31, 1982, pages 710-714 as an alpha-glucosidase inhibitor that reduced carbohydrate induced hypertriglyceridemia when is administered with the diet.

In ***Metabolism,*** Vol. 31, 1982, pages 88-92, it is reported that this compound was found capable of modifying the metabolic response of diabetic rats to high carbohydrate diet when it was included into such a diet.

## DESCRIPTION OF THE INVENTION

It has now been found that castanospermine possesses digestive enzyme inhibitory properties and thus can be used as an antidiabetic agent and as an inhibitor of increased lipid biosynthesis. Thus, when carbohydrate is ingested either as glucose or in a form such as maltose, sucrose or starch in food or drink, the blood glucose level rises to elevated concentrations. In healthy subjects, this hyperglycemic state quickly returns to normal, the glucose in the blood being rapidly metabolized and stored and/or utilized by the organism. In diabetes mellitus, however, the glucose tolerance of the patient is lowered and the abnormally high blood sugar levels which develop remain elevated for prolonged periods of time. A similar response to that seen in man can also be observed in other animals, including livestock, poultry, pet animals and laboratory animals. Such a condition can be described as postprandial hyperglycemia. One method for treating such a condition would be by administration of some substance which would prevent the conversion of complex sugars to glucose and thus prevent the development of the excessive glucose levels. In the present invention, it has been found that, where the high levels of glucose are a result of the hydrolysis of complex sugars, administration of castanospermine inhibits the initial formation of glucose in the blood and thus makes it possible to avoid the problems which would be associated with prolonged high levels of blood sugar.

Equivalent to castanospermine for the purposes of this invention are the salts of castanospermine with pharmaceutically acceptable acids and, particularly, with inorganic acids such as hydrochloric acid, hydrobromic acid, sulfuric acid and phosphoric acid; and with organic acids such as acetic acid, propionic acid, methanesulfonic acid and p-toluenesulfonic acid.

The mechanism whereby the above result is achieved is the following although the utility described above should not be limited by the precise details of this mechanism. Enzymes which catalyze the hydrolysis of complex carbohydrates convert non-absorbable carbohydrate into absorbable sugars. The rapid action of these enzymes leads to acute and undesirable elevations in blood glucose in diabetes. The compounds of the present invention are potent inhibitors of these enzymes and, when co-administered with a carbohydrate meal, they prevent harmful hyperglycemic excursions of this type.

This inhibition of the formation of glucose can be useful in other ways too. Thus, the carbohydrates ingested by an organism appear in the form of glucose and are metabolized into lipids such as triglycerides, cholesterol or phospholipids. Too large a carbohydrate intake results in increased biosynthesis of lipids, causing hyperlipemia and excessive accumulation of lipids, both in adipose tissue and in other systems in the organism which can lead to obesity, atherosclerosis, myocardial infarction and other kinds of heart diseases. The inhibition of the formation of glucose accomplished by castanospermine would thus be useful in the control of these other conditions.

The present Application also describes a process for the isolation of castanospermine from Castanospermum australe by a procedure that avoids the tedious extraction procedure and the use of the obnoxious solvent pyridine as described in the published procedure mentioned earlier while providing the desired product in greatly improved overall yields. Details of this procedure are set forth in the example.

The following test procedures can be used to demonstrate the activity of castanospermine.

Inhibitory Action Against Increase of Blood Glucose in Sucrose-Administered Mice

Male ICR-swiss mice with body weight of 25-35 g were fasted overnight. Sucrose was administered orally by gavage at a dose of 2 g/kg 15 minutes after the similar administration of castanospermine at doses of 0.125 to 60 mg/kg. Animals were sacrificed at 30 minutes after sucrose administration and blood glucose levels were measured by a method using glucose dehydrogenase. The results are shown in Table 1 below. The increase in glucose in the blood could be significantly inhibited by administering castanospermine at a dose of 0.125 mg/kg or higher.

TABLE 1

| Dose of Castanospermine | Number of Mice Tested | Blood Glucose Concentration (mg%) at 30 Min. After Sucrose |
|---|---|---|
| 0 mg/kg | 8 | 205 |
| 0.125 | 4 | 159 |
| 0.25 | 4 | 167 |
| 0.5 | 4 | 153 |
| 1.0 | 4 | 146 |
| 2.0 | 4 | 116 |
| Blank | 4 | 107 |
| (Blank: 0.5% methocel alone was administered.) | | |

Inhibitory Action Against Increase of Blood Glucose in Starch-Administrated Mice

Male ICR-Swiss mice with body weight of 25-35 g were fasted overnight. Starch was administered orally by gavage at a dose of 1 g/kg 15 minutes after the similar administration of castanospermine at doses of 1.9 to 60 mg/kg. Animals were sacrificed at 45 minutes after starch administration and blood glucose levels were measured by a method using glucose dehydrogenase. The results are shown in Table 2 below. The increase in glucose in the blood could be significantly inhibited by administering castanospermine at a dose of 3.8 mg/kg or higher.

TABLE 2

| Dose of the Compound of This Invention | Number of Mice Tested | Blood Glucose Concentration (mg%) at 45 min. After Starch |
|---|---|---|
| 0 mg/kg | 8 | 126 |
| 1.9 | 3 | 139 |
| 3.8 | 4 | 83 |
| 7.5 | 4 | 85 |
| Blank | 4 | 72 |
| (Blank: 0.5% methocel alone was administered.) | | |

In practicing this invention, an amount of castanospermine effective to inhibit postprandial hyperglycemia is administered to a mammal in need thereof by a suitable route. For the purposes of this invention, oral administration is preferred.

The effective amount of the compound, that is, the amount sufficient to inhibit postprandial hyperglycemia, depends on various factors such as the size, type and age of the animal to be treated, the particular compound or pharmaceutically acceptable salt employed, the frequency of administration, the severity of the condition and the time of administration. Generally speaking, the compounds would be administered orally at a dose of 10 mg to 500 mg at mealtime, with a dose of 40 mg to 200 mg being preferred. More specifically, the present compounds would be administered to humans in single unit doses (individual capsules) containing 50 mg of active ingredient with the material being administered three times a day at mealtime.

In practicing this invention, the active ingredient can be incorporated in a composition comprising a pharmaceutical carrier and from about 5 to about 90 percent by weight of castanospermine or a pharmaceutically-acceptable salt thereof. The term "pharmaceutical carrier" refers to known pharmaceutical excipients useful in formulating pharmaceutically active compounds for internal administration to animals, and which are substantially nontoxic and non-sensitizing under conditions of use. The compositions can be prepared by known techniques for the preparation of tablets, capsules, elixirs, syrups, emulsions, dispersions and wettable and effervescent powders, and can contain suitable excipients known to be useful in the preparation of the particular type of composition desired. Suitable pharmaceutical carriers and formulation techniques are found in standard texts, such as Remington's Pharmaceutical Sciences, Mack Publishing Company, Easton, Pennsylvania.

The following example is presented to illustrate the present invention.

EXAMPLE 1

Commercially available mature seeds of Castanospermum australe (300 g, wet weight) were ground and continuously extracted for 24 hours in a Soxhlet apparatus with 1.0 l of a 3:7 (v/v) mixture of water and 2-propanol. The extract was concentrated in vacuo to a final volume of 250 ml and was filtered. After extraction with petroleum ether (10 x 100 ml), the aqueous extract was subjected to column chromatography with Dowex 50W-X4 ($H^+$) ion-exchange resin (2.5 cm x 32 cm). The column was washed with distilled water (200 ml) and the desired alkaloid was eluted with 400 ml of 2 N aqueous ammonium hydroxide. The basic eluate was concentrated in vacuo to 125 ml and applied to another column containing Dowex 1-X4-($OH^-$) ion-exchange resin (2.5 cm x 20 cm). The column was eluted with 125 ml of water and the eluate was concentrated to a final volume of 30 ml. Trituration of the concentrate with 300 ml of acetone yielded 3.5 g of castanospermine as colorless crystals (1.2%, calculation on the basis of the weight of fresh seeds). The isolated crystals had the following physical properties:

mp: 212-225 °C

| Elemental analysis: | | | |
|---|---|---|---|
| Found: | C, 50.53%; | H, 8.04%; | N, 7.26%. |
| Calc for $C_8H_{15}NO_4$: | C, 50.79%; | H, 7.94%; | N, 7.41%. |

Optical rotation = $[\alpha]_D^{25}$ + 75.5 ° (c 1.72, $H_2O$)

Mass Spectral Data: 190 ($MH^+$), 172 ($MH^+$-$H_2O$).

## Claims

1. A composition for use in treating diabetes which comprises an effective amount of castanospermine.

2. A composition according to claim 1 for use in treating postprandial hyperglycemia in diabetic individuals which comprises an effective amounts of castanospermine.

3. A composition according to claim 1 for use in inhibiting carbohydrate absorption in diabetics which comprises an effective amount of castanospermine.

4. Castanospermine, i.e., 1S, 6S, 7R, 8R, 8aR - 1, 6, 7, 8 - tetrahydroxyindolizidine of formula

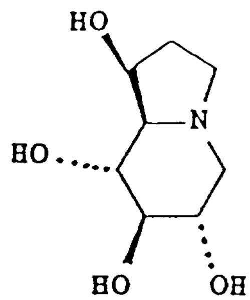

or a salt thereof with a pharmaceutically acceptable acid for use as a medicine.

5. Use of a compound of claim 4 for the preparation of a medicament for treating diabetes.

6. Use of a compound of claim 4 for the preparation of a medicament for treating postprandial hyperglycemia.

7. Use of a compound of claim 4 for the preparation of a medicament for inhibiting carbohydrate absorption.

## Patentansprüche

1. Mittel zur Verwendung für die Behandlung von Diabetes, umfassend eine wirksame Menge von Castanospermin.

2. Mittel nach Anspruch 1 zur Verwendung für die Behandlung von postprandialer Hyperglykämie in diabetischen Personen, umfassend eine wirksame Menge von castanospermin.

3. Mittel nach Anspruch 1 zur Verwendung für die Hemmung von Kohlenhydratabsorption in diabetischen Personen, umfassend eine wirksame Menge von castanospermin.

4. Castanospermin, nämlich 1S, 6S, 7R, 8R, 8aR -1,6,7,8-Tetrahydroxyindolizidin der Formel

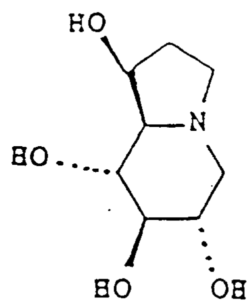

oder ein Salz davon mit einer pharmazeutisch verträglichen Säure zur Verwendung als Medikament.

5. Verwendung einer Verbindung nach Anspruch 4 für die Herstellung eines Medikaments zur Behandlung von Diabetes.

6. Verwendung einer Verbindung nach Anspruch 4 für die Herstellung eines Medikaments zur Behandlung von post-prandialer Hyperglykämie.

7. Verwendung einer Verbindung nach Anspruch 4 für die Herstellung eines Medikaments für die Hemmung von Kohlenhydratabsorption.

**Revendications**

1. Composition à utiliser dans le traitement du diabète, qui comprend une quantité efficace de castanospermine.

2. Composition selon la revendication 1, à utiliser dans le traitement de l'hyperglycémie postprandiale chez des individus diabétiques, qui comprend une quantité efficace de castanospermine.

3. Composition selon la revendication 1, à utiliser pour inhiber l'absorption des hydrates de carbone chez les diabétiques, qui comprend une quantité efficace de castanospermine.

4. Castanospermine, c'est-à-dire 1S, 6S, 7R, 8R, 8aR- 1,6,7,8-tétrahydroxyindolizidine de formule

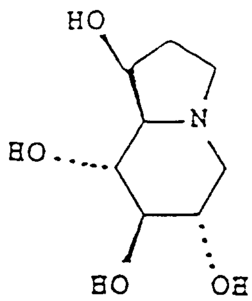

ou un de ses sels avec un acide pharmaceutiquement acceptable, à utiliser comme médicament.

5. Utilisation d'un composé de la revendication 4 pour la préparation d'un médicament pour le traitement du diabète.

6. Utilisation d'un composé de la revendication 4 pour la préparation d'un médicament pour le traitement de l'hyperglycémie postprandiale.

7. Utilisation d'un composé de la revendication 4 pour la préparation d'un médicament pour inhiber l'absorption des hydrates de carbone.